# EUROPÄISCHE PATENTSCHRIFT

(11) **EP 2 851 734 B1**
(45) Veröffentlichungstag und Bekanntmachung des Hinweises auf die Patenterteilung: **04.01.2023**
(21) Anmeldenummer: 14184574.3
(22) Anmeldetag: 12.09.2014
(51) Int. Cl.: A61B 34/30, G02B 25/00, G02B 27/00

(54) **Okular**
Eyepiece
Ocular

(30) Priorität: 20.09.2013 DE 102013110425
(43) Veröffentlichungstag der Anmeldung: 25.03.2015
(73) Patentinhaber: Karl Storz SE & Co. KG, 78532 Tuttlingen (DE); Storz Endoskop Produktions GmbH, 78532 Tuttlingen (DE)
(72) Erfinder: Krattiger, Beat, 78532 Tuttlingen (DE)

(56) Entgegenhaltungen:
- WO-A1-2013/076994
- WO-A2-03/083556
- WO-A2-2005/040866
- DE-A1- 3 708 633
- DE-U1-202007 011 682
- JP-A- 2010 049 111
- US-A- 4 217 048
- US-A- 5 835 289
- US-A1- 2013 173 029

## Beschreibung

Die vorliegende Erfindung ist auf ein Endoskop mit einem Okular, bezogen.

Okulare für medizinische und nicht medizinische Anwendungen ermöglichen in der Regel eine Positionierung bzw. Verschiebung der vom entspannten menschlichen Auge als scharf wahrgenommenen Fläche und eine Korrektur einer Achsen-Ametropie (insbesondere Hyperopie bzw. Weitsichtigkeit und Myopie bzw. Kurzsichtigkeit) eines Auges eines Verwenders des Okulars. Beides zusammen wird gemeinhin als Fokussierung bezeichnet und erfolgt in der Regel durch Verschiebung des gesamten Okulars oder von Teilen des Okulars parallel zur optischen Achse.

Die Augen sehr vieler Menschen weisen jedoch neben einer Achsen-Ametropie einen Astigmatismus auf. Aufgrund einer nicht-rotationssymmetrischen Krümmung der Hornhaut, einer nicht-rotationssymmetrischen Gestalt der Linse, einer ungleichförmigen Kontraktion des Ziliarmuskels und/oder aus einem anderen Grund werden von einem betrachteten punktförmigen Objekt ausgehende Lichtstrahlen nicht in einem Punkt, sondern in einer Linie auf der Netzhaut abgebildet. Insbesondere die Hornhaut und die Linse des Auges weisen in unterschiedlichen Richtungen bzw. in unterschiedlichen Ebenen senkrecht zur optischen Achse unterschiedliche Brechwerte auf. Die Differenz des maximalen Brechwerts und des minimalen Brechwerts wird in Dioptrien angegeben. Zur Charakterisierung eines Astigmatismus wird ferner die Orientierung der Ebene maximaler Brechkraft oder die Orientierung der Ebene minimaler Brechkraft angegeben, beide Achsen sind beim deutlich weiter verbreiteten regulären Astigmatismus orthogonal zueinander.

Personen mit nicht nur geringem Astigmatismus bevorzugen es in der Regel, ihre Brille auch beim Blick in ein Okular nicht abzunehmen. Dies setzt jedoch eine geeignete Konstruktion des Okulars voraus. Außerdem kann bei der nahezu unvermeidlichen Berührung mit dem Okular die Brille verschmutzt oder ihre oft hochwertige, aber mechanisch empfindliche Beschichtung beschädigt werden. Als Alternative stehen zu manchen Okularen einsetzbare oder ansetzbare Zylinderlinsen zur Verfügung. Das Okular ist dann allerdings nur noch durch eine Person oder Personen, die zufällig den gleichen Astigmatismus aufweisen, und auch für diese nur ohne Brille verwendbar. Wenn ein Okular durch unterschiedliche Personen oder abwechselnd mit und ohne Brille verwendet werden soll, kommen diese Zusatzlinsen aus praktischen Gründen nicht in Betracht.

In JP 2010-49111 ist ist ein Okular 4 für ein Operationsmikroskop 1 beschrieben. Zur Korrektur eines Astigmatismus eines Auges eines Operateurs kann an dem dem Auge zugewandten Ende des Okulars 4 eine Fassung mit einer Korrekturlinse 12 eingeschraubt werden.

In DE 20 2007 011 682 U1 ist eine Visiereinrichtung für eine Waffe beschrieben. Die Visiereinrichtung umfasst zwei Zylinderlinsen 1, 2, die relativ zu einander und gemeinsam um die optische Achse 5 rotierbar sind, um eine stufenlose Korrektion sowohl der Stärke als auch der Achsenlage eines Astigmatismus zu ermöglichen.

In WO 2005/040866 A2 ist eine Abbildungsoptik mit einstellbarer Brechkraft beschrieben. Eine Linse mit veränderbarer Brechkraft umfasst zwei nicht mischbare Flüssigkeiten 25, 27 mit unterschiedlichen Brechungsindizes. Gestalt und Krümmung der Grenzfläche zwischen beiden nicht mischbaren Flüssigkeiten 25, 27 können durch Anlegen einer elektrischen Spannung geändert werden.

Eine Aufgabe der vorliegenden Erfindung besteht darin, ein verbessertes Endoskop und eine verbesserte Steuerungsvorrichtung für einen Operationsroboter zu schaffen, die insbesondere auch durch medizinisches Personal mit Astigmatismus verwendbar sind.

Diese Aufgabe wird durch die Gegenstände der unabhängigen Ansprüche gelöst.

Weiterbildungen sind in den abhängigen Ansprüchen angegeben.

Das im beanspruchten Endoskop verwendete Okular umfasst eine Fokussiereinrichtung zur Positionierung der vom entspannten menschlichen Auge als scharf wahrgenommenen Fläche und zur Korrektur einer Achsen-Ametropie eines Auges eines Verwenders des Okulars, und eine Korrektureinrichtung zur einstellbaren Korrektur eines Astigmatismus eines Auges eines Verwenders des Okulars.

Die Fokussiereinrichtung ist insbesondere zur Verschiebung der ebenen oder gekrümmten Fläche, innerhalb derer Gegenstände vom entspannten menschlichen Auge als scharf wahrgenommen bzw. scharf auf die Retina abgebildet werden, und zur Korrektur einer Hyperopie bzw. Weitsichtigkeit oder Myopie bzw. Kurzsichtigkeit ausgebildet.

Die Fokussiereinrichtung umfasst insbesondere einen Gewinde- oder Spindeltrieb oder eine andere Einrichtung zum manuellen oder motorischen Bewegen des gesamten Okulars oder von Teilen desselben in Richtung parallel zur optischen Achse des Okulars.

Die Korrektureinrichtung ist insbesondere eine Einrichtung zur Einstellung eines zylindrischen Brechwerts des Okulars bzw. einer Differenz zwischen einem maximalen Brechwert und einem minimalen Brechwert des Okulars und zur Einstellung der Orientierung der Ebene maximalen Brechwerts. Die Korrektureinrichtung verbleibt beim Einstellen des zylindrischen Brechwerts bzw. beim Einstellen der Korrektur eines Astigmatismus immer vollständig am Okular. Die Einstellung des zylindrischen Brechwerts bzw. deren Korrektur erfolgt also nicht durch Austausch oder Einsetzen oder Ansetzen oder Abnehmen oder Herausnehmen einer Linse oder einer anderen lichtbrechenden Einrichtung. Die Korrektur bzw. der zylindrische Brechwert ist insbesondere stufenlos einstellbar. Die Korrektureinrichtung umfasst insbesondere eine oder mehrere zur Einstellung der Korrekturwirkung bzw. des zylindrischen Brechwerts bewegbare Linsen oder andere optischen Elemente.

Die Korrektureinrichtung ermöglicht eine einfache und schnelle Anpassung des Okulars an einen Astigmatismus eines Verwenders. Eine Beschaffung und Bevorratung sowie ein Einsetzen, Ansetzen, Entnehmen, Abnehmen oder Austauschen einer Zusatzlinse oder dergleichen ist nicht erforderlich. Das Okular kann von mehreren Personen mit unterschiedlichem Astigmatismus und von einer Person abwechselnd mit und ohne Brille verwendet werden, dabei ist nur jeweils eine neue Einstellung der Korrekturwirkung erforderlich.

Bei dem im beanspruchten Endoskop verwendeten Okular, wie es hier beschrieben ist, umfasst die Korrektureinrichtung zwei lichtbrechende Einrichtungen, die jeweils in unterschiedlichen Ebenen unterschiedliche Brechwerte aufweisen, und deren Abstand veränderbar ist, oder die relativ zueinander rotierbar sind.

Jede der zwei oder mehr lichtbrechenden Einrichtungen umfasst insbesondere eine oder mehrere Zylinderlinsen oder andere Linsen Linsengruppen oder Linsensysteme, die bezogen auf unterschiedliche Ebenen unterschiedliche Brechwerte aufweisen. Jede der zwei oder mehr lichtbrechenden Einrichtungen kann achromatisch oder apochromatisch ausgebildet sein, und dazu insbesondere je zwei oder mehr transparente Körper aus unterschiedlichen Materialien aufweisen. Eine achromatische oder apochromatische Ausgestaltung der Korrektureinrichtung ist ferner dadurch möglich, dass die zwei lichtbrechenden Einrichtungen unterschiedliche Materialien mit insbesondere im Wesentlichen gegenläufiger Abhängigkeit des Brechungsindex von der Wellenlänge aufweisen.

Der Abstand der beiden lichtbrechenden Einrichtungen der Korrektureinrichtung sind insbesondere durch Bewegen bzw. Verfahren von einer oder von beiden lichtbrechenden Einrichtungen in Richtung parallel zur optischen Achse des Okulars veränderbar. Im Fall einer Korrektureinrichtung, die drei oder mehr lichtbrechende Einrichtungen umfasst, können die Abstände mehrerer lichtbrechender Einrichtungen der Korrektureinrichtung durch Verfahren bzw. Bewegen von einer, zwei oder mehr lichtbrechenden Einrichtungen in Richtung parallel zur optischen Achse des Okulars verändert werden. Alternativ ist die zylindrische Korrekturwirkung bzw. der zylindrische Brechwert durch Rotation von einer oder mehreren der lichtbrechenden Einrichtungen um eine Achse einstellbar, wobei die Achse insbesondere parallel zur optischen Achse des Okulars ist.

Das im beanspruchten Endoskop verwendete Okular, wie es hier beschrieben ist, umfasst insbesondere eine Zylinderlinse mit positivem und eine Zylinderlinse mit negativem Brechwert. Die Beträge der Brechwerte können gleich oder unterschiedlich sein. Insbesondere weisen die beiden der jeweils anderen Zylinderlinse zugewandten Oberflächen der Zylinderlinsen gleiche Krümmungsradien auf, wobei eine der beiden Oberflächen konvex und die andere konkav ist. Der zylindrische Brechwert D der Kombination beider Zylinderlinsen beträgt näherungsweise D = (n-1)^{2 ∗} d/R². Dabei ist n der identische Brechungsindex beider Zylinderlinsen, d der Abstand der beiden Zylinderlinsen und R der Krümmungsradius der einander zugewandten zylindrischen Oberflächen der Zylinderlinsen. Diese Gleichung gilt für zwei Zylinderlinsen, deren voneinander abgewandte Seiten eben bzw. plan sind. Abweichend von diesem einfachen Fall können eine oder beide voneinander abgewandte Oberfläche zylindrisch oder anders gekrümmt sein. Anstelle zweier Zylinderlinsen sind also andere Linsen mit einander zugewandten zylindrischen Oberflächen verwendbar.

Eine komplementäre Ausgestaltung der einander zugewandten Oberflächen zweier gegeneinander verfahrbarer Linsen ermöglicht einen verschwindenden zylindrischen Brechwert, wenn die beiden komplementären Oberflächen einander berühren. Für eine achromatische oder apochromatische Ausgestaltung können die beiden Linsen unterschiedliche Materialien mit insbesondere im Wesentlichen gegenläufiger Abhängigkeit der Brechungsindizes von der Wellenlänge aufweisen.

Alternativ können die zwei lichtbrechenden Einrichtungen der Korrektureinrichtung zwei dem Betrage nach unterschiedliche Brechwerte aufweisen. In diesem Fall kann der zylindrische Brechwert der Korrektureinrichtung bei einem mittleren Abstand der zwei lichtbrechenden Einrichtungen verschwinden und von diesem mittleren Abstand ausgehend bei kleineren und größeren Abständen unterschiedliche Vorzeichen aufweisen.

Unterschiedliche Brechwerte sind beispielsweise bei gleichem Material der zwei lichtbrechenden Einrichtungen durch unterschiedliche Krümmungsradien realisierbar, oder bei gleichen Krümmungsradien durch zwei verschiedene Materialien mit unterschiedlichen Brechungsindizes.

Bei dem im beanspruchten Endoskop verwendeten Okular, wie es hier beschrieben ist, weist die Korrektureinrichtung insbesondere eine elektrisch, magnetisch oder mittels hydrostatischen Drucks formbare Grenzfläche zwischen zwei Medien mit unterschiedlichen Brechungsindizes auf.

Die Grenzfläche kann aufgrund unterschiedlicher Eigenschaften der beiden Medien, die eine Mischung verhindern, ausgebildet sein. Beispielsweise ist ein Medium eine hydrophile bzw. lipophobe Flüssigkeit und das andere Medium eine hydrophobe bzw. lipophile Flüssigkeit. Alternativ kann ein Medium flüssig und das andere Medium gasförmig sein. Die Anordnung beider Medien kann durch unterschiedliche Benetzungseigenschaften verschiedener Oberflächenbereiche einer Kammer, die die beiden Medien enthält, vorgegeben sein. Die Grenzfläche zwischen den beiden Medien ist insbesondere aufgrund unterschiedlicher elektrischer und/oder magnetischer Eigenschaften der beiden Medien durch ein elektrisches und/oder magnetisches Feld formbar. Ein elektrisches oder magnetisches Feld ist beispielsweise mittels Elektroden bzw. Spulen innerhalb oder außerhalb einer Kammer, innerhalb derer die beiden Medien angeordnet sind, erzeugbar.

Alternativ wird die Grenzfläche durch eine optisch transparente elastische Membran gebildet. Dabei ist insbesondere eines der beiden Medien flüssig und das andere Medium flüssig oder gasförmig. Die optisch transparente elastische Membran wird insbesondere verformt, indem die Differenz der hydrostatischen Drücke in beiden Medien verändert wird.

Durch eine nicht-rotationssymmetrische Ausgestaltung, beispielsweise in einer Ebene senkrecht zur optischen Achse des Okulars länglich-rechteckige Ausgestaltung der Anordnung der beiden Medien kann eine zumindest in einem mittleren Bereich im Wesentlichen zylindrische Gestalt der Grenzfläche zwischen den beiden Medien erzeugt werden. Die Grenzfläche zwischen den beiden Medien wirkt wie eine Zylinderlinse mit einstellbarer Krümmung.

Insbesondere eine elektrische oder magnetische Beeinflussung der formbaren Grenzfläche zwischen den beiden Medien ermöglicht eine einfache und robuste Steuerung des zylindrischen Brechwerts, ohne einen Lineartrieb oder eine andere Antriebseinrichtung mit beweglichen Teilen zu erfordern.

Bei dem im beanspruchten Endoskop verwendeten Okular, wie es hier beschrieben ist, ist die Korrektureinrichtung insbesondere um die optische Achse des Okulars rotierbar.

Insbesondere ist das gesamte Okular um die optische Achse rotierbar. Alternativ sind beispielsweise die erwähnten Zylinderlinsen oder ein Behältnis, in dem die erwähnten beiden Medien mit unterschiedlichen Brechungsindizes angeordnet sind, um die optische Achse des Okulars rotierbar.

Das im beanspruchten Endoskop verwendete Okular, wie es hier beschrieben ist, umfasst insbesondere ferner eine Bedieneinrichtung zum Einstellen eines zylindrischen Brechwerts.

Die Bedieneinrichtung ist beispielsweise ringförmig und symmetrisch zur optischen Achse des Okulars angeordnet. Die Bedieneinrichtung ist insbesondere um die optische Achse des Okulars rotierbar und durch einen Gewinde- bzw. Spindeltrieb mit der Korrektureinrichtung gekoppelt.

Das im beanspruchten Endoskop verwendete Okular, wie es hier beschrieben ist, umfasst ferner insbesondere eine weitere Bedieneinrichtung zum Einstellen der Orientierungen maximalen und minimalen Brechwerts der Korrektureinrichtung.

Die weitere Bedieneinrichtung ist beispielsweise ringförmig und symmetrisch zur optischen Achse des Okulars angeordnet und um die optische Achse des Okulars rotierbar und mit der Korrektureinrichtung mittelbar oder unmittelbar mechanisch verbunden.

An der Bedieneinrichtung und an der weiteren Bedienrichtung sind jeweils insbesondere Skalen für den zylindrischen Brechwert und für einen die Orientierungen maximalen und minimalen Brechwerts beschreibenden Winkel angeordnet.

Bei dem im beanspruchten Endoskop verwendeten Okular, wie es hier beschrieben ist, bildet insbesondere die Korrektureinrichtung eine proximale Lichtaustrittsfläche des Okulars.

Die Korrektureinrichtung bildet insbesondere ein proximales Deckglas bzw. ein das Okular proximal abschließendes Fensterbauteil. Anders ausgedrückt ist das Deckglas bzw. das Fensterbauteil Teil der Korrektureinrichtung. Dazu ist beispielsweise eine der oben erwähnten Zylinderlinsen oder ein Lichtaustrittsfenster eines Behältnisses, in dem zumindest eines der beiden oben erwähnten Medien mit unterschiedlichen Brechungsindizes angeordnet ist, gleichzeitig als Lichtaustrittsfenster des Okulars ausgebildet. Dies ermöglicht eine sehr weit proximale Anordnung der Korrektureinrichtung und somit eine insgesamt sehr kompakte Bauform des Okulars.

Das im beanspruchten Endoskop verwendete Okular, wie es hier beschrieben ist, umfasst ferner insbesondere eine motorische Antriebseinrichtung zum motorischen Einstellen der Korrektureinrichtung.

Das im beanspruchten Endoskop verwendete Okular umfasst insbesondere eine motorische Antriebseinrichtung zum Einstellen des zylindrischen Brechwerts und eine motorische Antriebseinrichtung zum Einstellen der Orientierungen des maximalen und des minimalen Brechwerts. Eine motorische Antriebseinrichtung umfasst insbesondere einen Schrittmotor oder einen anderen Elektromotor, einen Ultraschallmotor oder einen Piezomotor.

Eine motorische Antriebseinrichtung kann eine schnelle Einstellung der Korrekturwirkung der Korrektureinrichtung des Okulars ermöglichen. Die motorischen Antriebseinrichtungen können mit einer Kamera und einer Einrichtung zur Gesichts-, Iris- oder Retinaerkennung, einem RFID-Empfänger oder einer anderen Identifizierungseinrichtung sowie einer Datenbank, in der Parameter abgelegt sind, die den Astigmatismus und optional weitere Ametropien von einem oder mehreren Benutzern beschreiben, gekoppelt sein. Dies kann eine automatische Anpassung des Okulars an einen Benutzer ermöglichen, sobald dieser sich dem Okular nähert.

Ein Endoskop umfasst ein Okular, wie es hier beschrieben ist.

Ein Endoskop mit einem Okular, wie es hier beschrieben ist, weist insbesondere einen Astigmatismus auf, der von einem Brechungsindex eines Mediums, in dem das distale Ende des Endoskops angeordnet ist, abhängig ist.

Insbesondere medizinische Endoskope werden oft abwechselnd in Luft, Kohlenstoffdioxid oder einem anderen Gas, das einen Brechungsindex nahe 1 aufweist, und in Wasser oder einer anderen wässrigen Flüssigkeit betrieben. Insbesondere wenn das distale Lichteintrittsfenster des Endoskops nicht-sphärisch ist, beispielsweise die Gestalt eines Ausschnitts eines Kreiszylinders aufweist, resultiert ein vom Brechungsindex des Mediums, in dem das distale Lichteintrittsfenster angeordnet ist, abhängiger Astigmatismus des Endoskops.

Die Korrektureinrichtung des Okulars kann auch eine Korrektur des vom Brechungsindex des Mediums abhängigen Astigmatismus des Endoskops ermöglichen.

Das beanspruchte Endoskop mit einem vom Brechungsindex eines Mediums abhängigen Astigmatismus umfasst ferner eine Bedieneinrichtung zum Andern des zylindrischen Brechwerts der Korrektureinrichtung um einen vorbestimmten Betrag, der dem Unterschied zwischen dem Astigmatismus des Endoskops bei Anordnung des distalen Endes in Luft und dem Astigmatismus des Endoskops bei Anordnung des distalen Endes in Wasser entspricht.

Die Bedieneinrichtung ermöglicht ein Umschalten der Korrekturwirkung der Korrektureinrichtung zwischen den beiden bei Anordnung des distalen Endes des Endoskops in Luft bzw. in Wasser erforderlichen Korrekturwirkungen. Einstellungen am Okular, die auf den Astigmatismus des Auges eines Benutzers bezogen sind, können also erhalten bleiben.

Eine Steuerungsvorrichtung für einen Chirurgieroboter umfasst ein Okular, wie es hier beschrieben ist.

Ein Chirurgieroboter ist eine Vorrichtung zum Durchführen medizinischer, insbesondere chirurgischer Eingriffe mittels Servomotoren. Bewegungen von Instrumenten und Werkzeugen oder des Chirurgieroboters selbst werden nicht unmittelbar oder mittelbar manuell angetrieben, sondern von Servomotoren erzeugt. Der Chirurgieroboter und seine Antriebseinrichtungen werden von medizinischem Personal gesteuert, wobei ein Computer die vom chirurgischen Personal erzeugten Steuerbefehle aufbereiten, filtern, hinsichtlich der Einhaltung von Sicherheitsregeln prüfen kann bevor die Steuersignale oder modifizierte Steuersignale an die Antriebseinrichtungen des Chirurgieroboters weitergeleitet werden. Die Steuerung eines Chirurgieroboters setzt in der Regel ein hohes Maß an Konzentration voraus. Um störende optische Einflüsse vom medizinischen Personal fernzuhalten, und um dem medizinischen Personal einen dreidimensionalen Eindruck vom Operationsfeld zu vermitteln, können an einer Steuervorrichtung für einen Chirurgieroboter zwei Okulare vorgesehen sein.

Eine Steuerungsvorrichtung, wie sie hier beschrieben ist, umfasst insbesondere eine Ablageeinrichtung zum Aufnehmen einer Brille. Medizinisches Personal mit Ametropie, das das Operationsmikroskop bzw. die Steuerungsvorrichtung verwendet, kann seine Brille unmittelbar am an der Steuerungsvorrichtung ablegen.

### Kurzbeschreibung der Figuren

Nachfolgend werden Ausführungsformen anhand der beigefügten Figuren näher erläutert. Es zeigen:
Figur 1 eine schematische Darstellung eines Endoskops;
Figur 2 eine schematische Darstellung eines im beanspruchten Endoskop verwendeten Okulars;
Figur 3 eine weitere schematische Darstellung des im beanspruchten Endoskop verwendeten Okulars aus Figur 2;
Figur 4 eine weitere schematische Darstellung des im beanspruchten Endoskop verwendeten Okulars aus den Figuren 2 und 3;
Figur 5 eine schematische Darstellung eines weiteren im beanspruchten Endoskop verwendeten Okulars;
Figur 6 eine schematische Darstellung optischer Einrichtungen eines weiteren im beanspruchten Endoskop verwendeten Okulars;
Figur 7 eine schematische Darstellung optischer Einrichtungen eines weiteren im beanspruchten Endoskop verwendeten Okulars;
Figur 8 eine schematische Darstellung eines nicht im Schutzbereich der Anmeldung liegenden Operationsmikroskops;
Figur 9 eine schematische Darstellung einer Steuerungsvorrichtung.

### Beschreibung der Ausführungsformen

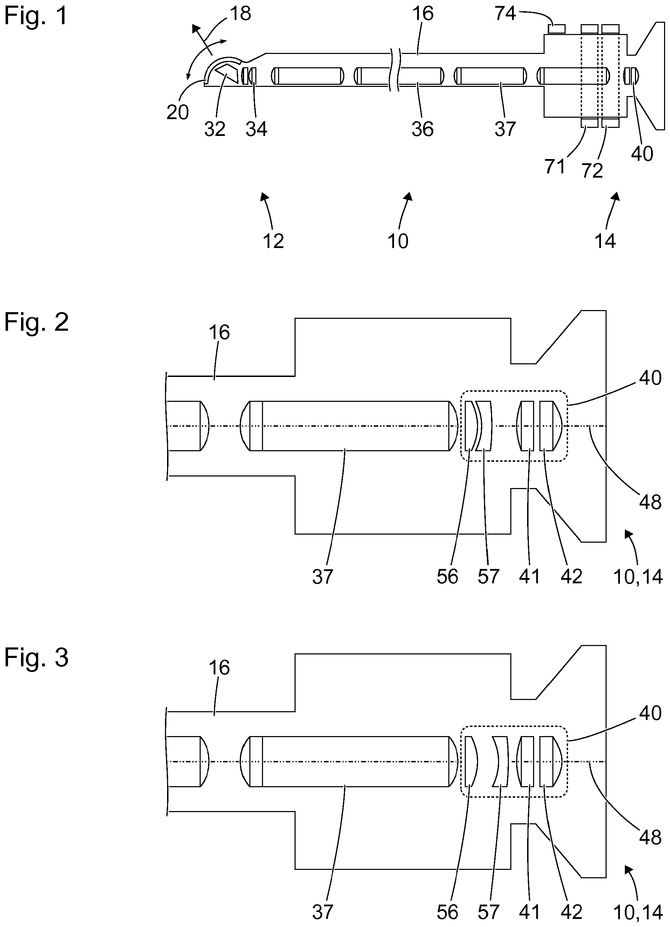

Figur 1 zeigt eine schematische Darstellung eines Endoskops 10 mit einem distalen Ende 12 und einem proximalen Ende 14. Ein gerader und starrer Schaft 16 erstreckt sich vom distalen Ende 12 bis zum proximalen Ende 14 des Endoskops 10. Die Blickrichtung 18 des Endoskops 10 ist innerhalb eines in Figur 1 durch einen gebogenen Doppelpfeil angedeuteten Winkelbereichs einstellbar.

Das Endoskop 10 und seine bisher beschriebenen Merkmale sind in Figur 1 im Wesentlichen durch Konturen angedeutet. Die nachfolgend beschriebenen optischen Einrichtungen sind hingegen in einer Seitenansicht angedeutet. Dabei sind nicht nur die äußeren Konturen der optischen Einrichtungen, wie sie bei einem Schnitt entlang einer Ebene, die die optische Achse enthält, sichtbar werden, sondern auch Kanten, die sich insbesondere kreisbogenförmig aus der Zeichenebene herauswölben, in Projektion auf die Zeichenebene dargestellt. Insbesondere sind kreisbogenförmige Kanten zwischen gewölbten Lichteintrittsflächen oder Lichtaustrittsflächen einerseits und zylindrischen Mantelflächen (parallel zur optischen Achse) andererseits als gerade Linien orthogonal zur Längsachse des Endoskops 10 erkennbar, da diese kreisbogenförmigen Kanten in Ebenen orthogonal zur optischen Achse und damit auch orthogonal zur Längsachse des Endoskops 10 und zur Zeichenebene liegen. Ferner sind als gerade Linien die ebenfalls kreisbogenförmigen Ränder von Grenzflächen zwischen miteinander verkitteten Elementen erkennbar.

Das Endoskop 10 umfasst am distalen Ende 12 ein Fensterbauteil 20 aus einem Material, das insbesondere für Licht innerhalb des für das menschliche Auge sichtbaren Wellenlängenbereichs transparent ist bzw. eine möglichst hohe Transmission aufweist. Das Fensterbauteil 20 weist die Gestalt eines Ausschnitts eines Kreiszylindermantels auf, wobei die Zylinderachse des Kreiszylindermantels orthogonal zur optischen Achse weiterer optischer Einrichtung des Endoskops 10, orthogonal zur Längsachse des Schafts 16, orthogonal zur Blickrichtung 18 des Endoskops und orthogonal zur Zeichenebene ist.

Unmittelbar proximal und lichtstromabwärts des Fensterbauteils 20 ist ein Schwenkprisma 32 angeordnet. Das Schwenkprisma 32 weist insbesondere die Gestalt eines Dove-Prismas auf und ist um eine nicht dargestellte Schwenkachse orthogonal zur Zeichenebene der Figur 1 schwenkbar, um die Blickrichtung 18 einzustellen.

Lichtstromabwärts des Schwenkprismas 32 ist ein Objektiv 34 aus einer oder mehreren Linsen oder eine andere Abbildungseinrichtung zur Erzeugung eines Zwischenbilds eines mittels des Endoskops 10 beobachteten Gegenstands angeordnet. Proximal und lichtstromabwärts des Objektivs 34 ist ein Stablinsensystem aus mehreren Stablinsen 36, 37 oder ein anderes Relaislinsensystem zur Übertragung des vom Objektiv 34 erzeugten Zwischenbilds zum distalen Ende 14 des Endoskops 10 im Schaft 16 angeordnet.

Proximal und lichtstromabwärts des Stablinsensystems 36, 37 ist ein Okular 40 zum Erzeugen eines virtuellen Bilds, das vom menschlichen Auge erfassbar ist, angeordnet. Das Okular 40 umfasst insbesondere mehrere Linsen und verschließt gleichzeitig das Endoskop 10 am proximalen Ende 14 hermetisch dicht.

Das Fensterbauteil 20 am distalen Ende 12 des Endoskops 10 erzeugt aufgrund seiner nicht-sphärischen, sondern kreiszylindermantelförmigen Gestalt Abbildungsfehler bzw. Aberrationen, die umso größer sind, je größer das Verhältnis zwischen der Wanddicke und dem Krümmungsradius des Fensterbauteils 20 ist und je größer der Durchmesser eines mittels des Schwenkprismas, des Objektivs 34, des Stablinsensystems 36, 37 und des Okulars 40 erfassten Lichtbündels ist. Insbesondere weist das Fensterbauteil 20 einen axialen Astigmatismus auf.

Das Okular 40 ist zur Korrektur eines Astigmatismus eines Auges eines Benutzers des Okulars ausgebildet. Dazu weist das Okular insbesondere eine Korrektureinrichtung und weitere Merkmale und Eigenschaften auf, die unten mit Bezug zu den Figuren 2 bis 7 näher beschrieben sind. Die Korrektureinrichtung ist insbesondere ferner zur Korrektur eines von der nicht zur Blickrichtung 18 rotationssymmetrischen Gestalt des Fensterbauteils 20 herrührenden Astigmatismus des Endoskops 10 geeignet und ausgebildet.

Am proximalen Ende 14 des Endoskops 10 sind Bedieneinrichtungen 71, 72, 74 vorgesehen, mittels derer das Okular 40 einstellbar ist. Insbesondere ist mittels einer ersten Bedieneinrichtung 71 ein zylindrischer Brechwert des Okulars 40 einstellbar. Insbesondere sind mittels einer zweiten Bedieneinrichtung 72 die Orientierungen der die optische Achse des Okular enthaltenden Ebenen, in denen das Okular einen maximalen bzw. einen minimalen Brechwert aufweist, einstellbar. Insbesondere ist die dritte Bedieneinrichtung 74 ausgebildet, um den zylindrischen Brechwert des Okulars 40 um einen Betrag zu ändern, der der Differenz der zylindrischen Brechwerte der Lichteintrittsfläche des Fensterbauteils in Luft und in Wasser entspricht.

Figur 2 zeigt eine schematische Schnittdarstellung eines Okulars 40 am proximalen Ende 14 eines in Figur 2 nur angedeuteten Endoskops 10. Die Zeichenebene und die Art der Darstellung, insbesondere die Art der Darstellung optischer Elemente, entsprechen denen der Figur 1. Das Endoskop 10 weist insbesondere Merkmale und Eigenschaften auf, die oben anhand der Figur 1 dargestellt sind.

Das Okular 40 ist am proximalen Ende 14 des Endoskops 10 angeordnet, um ein durch das menschliche Auge erfassbares virtuelles Bild eines durch eine Stablinse 37 erzeugten Zwischenbilds zu erzeugen. Das Okular 40 umfasst eine erste, distale Linse 41 und eine zweite, proximale Linse 42, die jeweils sphärisch oder zumindest rotationssymmetrisch zur optischen Achse 48 des Okulars 40 sind. Die zweite, proximale Linse 42 verschließt das Okular nach proximal hermetisch dicht. Alternativ ist zum hermetisch dichten Verschluss ein transparentes Fensterbauteil mit zwei planen und parallelen Oberflächen vorgesehen. Ein derartiges Fensterbauteil kann den Vorteil haben, dass es gegenüber der optischen Achse 48 des Okulars nicht justiert werden muss.

Ferner umfasst das Okular 40 eine Korrektureinrichtung mit einer ersten, distalen Korrekturlinse 56 und einer zweiten, proximalen Korrekturlinse 57. Jede Korrekturlinse 56, 57 weist jeweils in zwei verschiedenen Ebenen, die die optische Achse 48 des Okulars 40 enthalten, zwei verschiedene Brechwerte auf. Jede der beiden Korrekturlinsen 56, 57 ist insbesondere als Zylinderlinse ausgebildet.

Ein Zylinder ist ein Körper, der durch zwei ebene und parallele Flächen, die auch als Grund- und Deckfläche bezeichnet werden, und durch eine Mantel- bzw. Zylinderfläche begrenzt wird. Die Mantel- bzw. Zylinderfläche wird von parallelen Geraden, die einander entsprechende Punkte an den Rändern der ebenen Flächen verbinden, gebildet. Ein Zylinder entsteht also durch Verschiebung einer ebenen Fläche oder Kurve entlang einer Geraden, die nicht in der Ebene der Fläche bzw. Kurve liegt. Grund- und Deckfläche können kreisförmig sein oder eine beliebige andere Gestalt aufweisen. Wenn die Grundfläche und die Deckfläche kreisförmig sind, ist der Zylinder ein Kreiszylinder. Wenn die die Mantel- bzw. Zylinderfläche bildendenden Geraden senkrecht zu den ebenen Flächen sind, wird der Zylinder als gerader Zylinder bezeichnet.

Mit einer Zylinderachse eines Zylinders ist jede Gerade gemeint, die parallel zu den Geraden, die die Mantel- bzw. Zylinderfläche bilden, ist. Die Zylinderachse gibt die Richtung an, in der der Zylinder innerhalb gewisser Grenzen translationsinvariant ist. Die Zylinderachse eines geraden Kreiszylinders ist die Gerade, auf der die Mittelpunkte von Grund- und Deckfläche liegen. Der gerade Kreiszylinder ist rotationssymmetrisch zu dieser Symmetrieachse.

Bei einer Zylinderlinse spielen die ebenen und parallelen Grund- und Deckfläche des Zylinders keine Rolle und sind insbesondere durch Schleifen oder auf andere Weise entfernt oder verändert. Die Lichteintrittsfläche und die Lichtaustrittsfläche werden durch Teile der Mantelfläche eines Zylinders gebildet, dessen Zylinderachse insbesondere orthogonal ist zur optischen Achse der optischen Einrichtung, deren Teil die Zylinderlinse ist.

Die Korrekturlinsen 56, 57 sind insbesondere Zylinderlinsen, deren Zylinderachsen orthogonal zur optischen Achse 48 des Okulars 40 und orthogonal zur Zeichenebene der Figur 2 sind. Die Korrekturlinsen 56, 57 weisen (bezogen auf eine Ebene orthogonal zur optischen Achse 48) rechteckige Konturen auf.

Bei der in Figur 2 angedeuteten Ausgestaltung der Korrekturlinsen 56, 57 als Zylinderlinsen, deren Zylinderachsen orthogonal zur Zeichenebene der Figur 2 sind, weist jede Korrekturlinse 56, 57 in einer die optische Achse 48 des Okulars 40 enthaltenden Ebene senkrecht zur Zeichenebene der Figur 2 einen verschwindenden Brechwert (Brechwert = 0 Dpt.) auf.

Bei der in Figur 2 angedeuteten Ausgestaltung der Korrekturlinsen 56, 57 weisen die der zweiten Korrekturlinse 57 zugewandte Lichtaustrittsfläche der ersten Korrekturlinse 56 und die der ersten Korrekturlinse 56 zugewandte Lichteintrittsfläche der zweiten Korrekturlinse 57 die gleiche, insbesondere kreiszylindrische Gestalt auf. Die erste Korrekturlinse 56 ist konvex, die zweite Korrekturlinse 57 ist konkav. Wenn die einander zugewandten Oberflächen der Korrekturlinsen 56, 57 einander berühren, wie es in Figur 2 näherungsweise angedeutet ist, beträgt der Brechwert der beiden einander zugewandten Oberflächen der Korrekturlinsen 56, 57 null. Wenn die beiden einander zugewandten Oberflächen der Korrekturlinsen 56, 57 voneinander beabstandet sind, weisen sie zusammen einen zylindrischen Brechwert auf, der von null verschieden ist.

Die von der zweiten Korrekturlinse 57 abgewandte Lichteintrittsfläche der ersten Korrekturlinse 56 und die von der ersten Korrekturlinse 56 abgewandte Lichtaustrittsfläche der zweiten Korrekturlinse 57 können jeweils eben bzw. plan oder gekrümmt sein. In Figur 2 ist beispielhaft eine leichte zylindrische Krümmung der von der ersten Korrekturlinse 56 abgewandten Oberfläche der zweiten Korrekturlinse 57 angedeutet.

Abweichend von der Darstellung in Figur 2 können die von der zweiten Korrekturlinse 57 abgewandte Lichteintrittsfläche der ersten Korrekturlinse 56 und die von der ersten Korrekturlinse 56 abgewandte Lichtaustrittsfläche der zweiten Korrekturlinse 57 jeweils die Gestalt eines Ausschnitts eines Kreiszylinders, eines anderen Zylinders, einer Kugeloberfläche, einer Oberfläche eines Rotationsellipsoids, einer Oberfläche eines Torus oder eine andere Gestalt aufweisen.

Das gesamte Okular 40 ist mittels einer in Figur 1 nicht dargestellten Bedieneinrichtung und eines Spindeltriebs oder mittels einer anderen in den Figuren nicht dargestellten Einrichtung manuell oder motorisch in Richtung parallel zur optischen Achse 48 des Okulars 40 bewegbar. Alternativ sind eine oder beide sphärische Linsen 41, 42 des Okulars 40 mittels eines Spindeltriebs oder auf andere Weise manuell oder motorisch parallel zur optischen Achse 48 des Okulars 40 bewegbar. Durch eine geeignete Positionierung des gesamten Okulars 40 oder von einer oder beiden sphärischen Linsen 41, 42 des Okulars 40 kann ein vom menschlichen Auge durch das Okular 40 erfasstes bzw. auf der Retina des menschlichen Auges erzeugtes Bild scharfgestellt werden. Dabei wird insbesondere auch eine Achsen-Ametropie des Auges korrigiert.

Ein Astigmatismus des Auges des Verwenders des Endoskops 10 und ggf. ein Astigmatismus des Endoskops 10 sind durch eine Positionierung des gesamten Okulars 40 oder von einer oder beiden sphärischen Linsen 41, 42 allein noch nicht korrigierbar. Zur Korrektur eines Astigmatismus des Auges des Verwenders und/oder zur Korrektur eines Astigmatismus des Endoskops 10 ist der Abstand der Korrekturlinsen 56, 57 voneinander veränderbar, indem zumindest eine der beiden Korrekturlinsen 56, 57 parallel zur optischen Achse 48 des Okulars 40 bewegbar ist. Ferner sind beide Korrekturlinsen 56, 57 gemeinsam oder das gesamte Okular 40 um die optische Achse 48 rotierbar, um die Orientierungen der Ebenen maximalen und minimalen Brechwerts einzustellen.

Das Okular 40 und seine Bestandteile sind mit den in Figur 1 dargestellten Bedieneinrichtungen 71, 72, 74 mechanisch und/oder mittels Magneten, die eine Kopplung durch eine hermetisch dichte Hülle hindurch ermöglichen, gekoppelt. Insbesondere ist die erste Bedieneinrichtung 71 zum Einstellen der zylindrischen Brechkraft mit den Korrekturlinsen 56, 57 derart gekoppelt, dass der Abstand der Korrekturlinsen 56, 57 mittels der ersten Bedieneinrichtung 71 verändert werden kann. Die zweite Bedieneinrichtung 72 ist insbesondere derart mit den Korrekturlinsen 56, 57 gekoppelt, dass mittels der zweiten Bedieneinrichtung 72 die Korrekturlinsen 56, 57 oder das gesamte Okular 40 um die optische Achse 48 des Okulars 40 rotiert werden kann. Die optionale dritte Bedieneinrichtung 74 ist insbesondere als mechanischer Schalter ausgebildet oder zwischen zwei Positionen veschiebbar oder rotierbar, die beispielsweise durch Rastungen definiert sind. Die dritte Bedieneinrichtung 74 ist insbesondere derart mit den Korrekturlinsen 56, 57 gekoppelt, dass mittels der dritten Bedieneinrichtung 74 der zylindrische Brechwert der Korrektureinrichtungen 56, 57 um einen vorbestimmten Brechwert, der dem Unterschied des Astigmatismus des Endoskops 10 bei Anordnung des distalen Endes 12 (vgl. Figur 1) in Luft oder Wasser entspricht, geändert werden kann.

Figur 3 zeigt eine weitere schematische Darstellung des Okulars 40 aus Figur 2. Die Zeichenebene und die Art der Darstellung entsprechen denen der Figuren 1 und 2. Figur 3 zeigt das Okular 40 in einer Situation bzw. Konfiguration mit einem gegenüber der Situation bzw. Konfiguration in Figur 2 vergrößerten Abstand der Korrekturlinsen 56, 57. Während der zylindrische Brechwert der Kombination beider Korrekturlinsen 56, 57 bei der in Figur 2 dargestellten Konfiguration klein ist, ist er bei der in Figur 3 dargestellten Konfiguration größer. Durch Verschieben der zweiten Korrekturlinse 57 relativ zur ersten Korrekturlinse 56 sind viele verschiedene zylindrische Brechwerte innerhalb eines vorbestimmten Intervalls einstellbar.

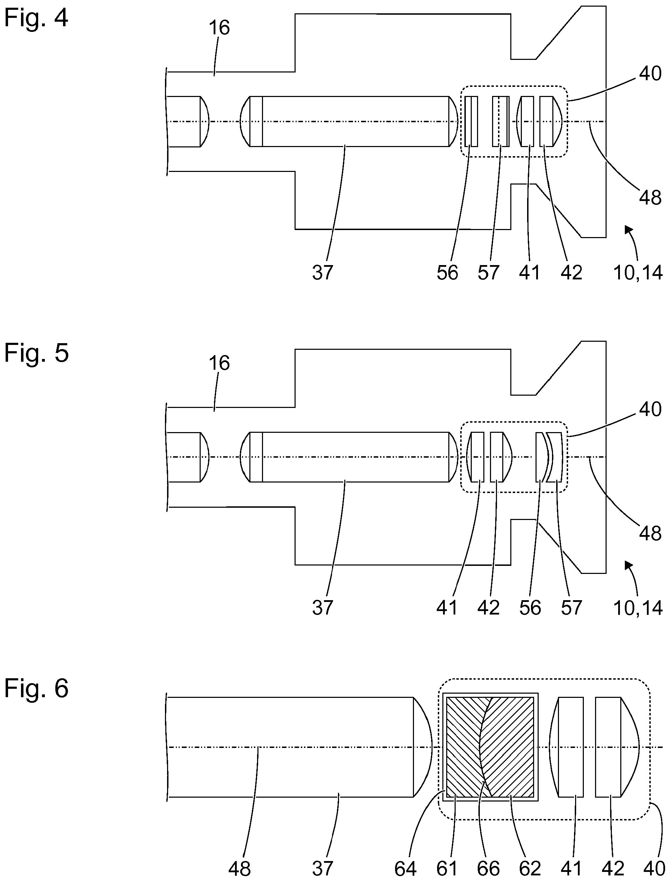

Figur 4 zeigt eine weitere schematische Darstellung des Okulars 40 aus den Figuren 2 und 3. Die Zeichenebene ist orthogonal zu den Zeichenebenen der Figuren 1 bis 3. Bezogen auf die Zeichenebene der Figur 4 beträgt der gemeinsame Brechwert beider einander zugewandter zylindrisch gekrümmter Oberflächen der Korrekturlinsen 56, 57 null. Der gemeinsame Brechwert der beiden einander zugewandten zylindrisch gekrümmten Oberflächen der Korrekturlinsen 56, 57 ist nur in den Zeichenebenen der Figuren 2 und 3 von Null verschieden und einstellbar.

Figur 5 zeigt eine schematische Darstellung eines weiteren Okulars 40 am proximalen Ende 14 eines in Figur 5 nur angedeuteten Endoskops 10. Die Zeichenebene und die Art der Darstellung entsprechen denen der Figuren 1 bis 3. Das Okular 40 ähnelt in einigen Merkmalen und Eigenschaften dem oben anhand der Figuren 2 bis 4 dargestellten Okular.

Nachfolgend sind Merkmale und Eigenschaften des Okulars 40 beschrieben, in denen sich dieses von dem oben anhand der Figuren 2 bis 4 dargestellten unterscheidet.

Das in Figur 5 dargestellte Okular unterscheidet sich von dem oben anhand der Figuren 2 bis 4 dargestellten Okular insbesondere dadurch, dass die beiden Korrekturlinsen 56, 57 proximal der beiden sphärischen Linsen 41, 42 angeordnet sind. Während bei dem anhand der Figuren 2 bis 4 dargestellten Okular 40 insbesondere die proximale sphärische Linse 42 das Okular 40 nach proximal hermetisch dicht verschließt, wird diese Aufgabe bei dem in Figur 5 dargestellten Okular insbesondere durch die proximale Korrekturlinse 57 übernommen.

Abweichend von den Darstellungen anhand der Figuren 2 bis 5 können insbesondere die beiden Korrekturlinsen 56, 57 zwischen den zwei sphärischen Linsen 41, 42 angeordnet sein. Abweichend von den Darstellungen anhand der Figuren 2 bis 5 können mehr als zwei sphärische Linsen und/oder mehr als zwei Korrekturlinsen 56, 57 vorgesehen sein.

Figur 6 zeigt eine schematische Darstellung eines weiteren Okulars 40, das in einigen Merkmalen und Eigenschaften den oben anhand der Figuren 2 bis 5 dargestellten Okularen ähnelt. Die Zeichenebene entspricht den Zeichenebenen der Figuren 1 bis 3 und 5, die Art der Darstellung entspricht derjenigen der Figuren 1 bis 5, wobei jedoch keine Konturen eines Endoskops angedeutet sind. Nachfolgend sind Merkmale und Eigenschaften des Okulars 40 beschrieben, in denen sich dieses von den oben anhand der Figuren 2 bis 5 dargestellten Okularen unterscheidet.

Das in Figur 6 dargestellte Okular 40 unterscheidet sich von den oben anhand der Figuren 2 bis 5 dargestellten Okularen insbesondere dadurch, dass anstelle zweier Korrekturlinsen, deren Abstand einstellbar ist, eine Flüssiglinse mit einem ersten Medium 61 und einem zweiten Medium 62 in einer Kammer 64 vorgesehen ist. Die Medien 61, 62 und zumindest die zum Lichtdurchtritt vorgesehenen Wandabschnitte der Kammer 64 sind optisch transparent. Die Medien 61, 62 weisen unterschiedliche Brechungsindizes auf, so dass an der Grenzfläche 66 zwischen den Medien 61, 62 Licht gebrochen wird.

Die Grenzfläche 66 zwischen den Medien 61, 62 kann durch eine optisch transparente und mechanisch elastische Membran gebildet sein. Alternativ kann die Grenzfläche 66 von einer geringen Mischbarkeit der beiden Medien 61, 62 herrühren. Beispielsweise ist eines der beiden Medien 61, 62 flüssig, das andere gasförmig oder eines der beiden Medien 61, 62 lipophil bzw. hydrophob und das andere hydrophil bzw. lipophob.

Insbesondere weisen beide Medien 61, 62 die gleiche oder eine ähnliche Massendichte auf. In diesem Fall beeinflussen die Gravitation und ggf. eine beschleunigte Bewegung die Medien 61, 62 nicht oder nur wenig, und die Gestalt der Grenzfläche 66 ist von der Orientierung des Okulars 40 im Gravitationsfeld der Erde nicht oder nur wenig abhängig und wird auch durch Erschütterungen nicht oder wenig verändert.

Die beiden Medien 61, 62 weisen unterschiedliche elektrische Eigenschaften, beispielsweise unterschiedliche Dielektrizitätskonstanten auf. An oder in der Kammer 64 sind in Figur 6 nicht dargestellte Elektroden zum Erzeugen elektrostatischer Felder vorgesehen, mittels derer die Gestalt der Grenzfläche 66 zwischen den Medien 61, 62 beeinflusst werden kann.

Alternativ weisen die Medien 61, 62 unterschiedliche magnetische Eigenschaften auf, und an der Kammer 64 sind eine oder mehrere Spulen zum Erzeugen eines Magnetfelds vorgesehen, mittels dessen die Gestalt der Grenzfläche 66 zwischen den Medien 61, 62 beeinflusst werden kann.

Alternativ oder zusätzlich steht die Kammer 64 mit einer oder mehreren Druckquellen oder Ausgleichsbehältern in fluidischer Verbindung, die eine Zufuhr oder Abfuhr eines oder beider Medien 61, 62 aus der Kammer bzw. in die Kammer 64 zur Formung der Grenzfläche 66 ermöglichen.

Wenn die Grenzfläche 66 zwischen den beiden Medien 61, 62 durch eine Membran gebildet ist, ist diese Membran insbesondere in eine Richtung versteift oder weist aus anderen Gründen anisotrope elastische Eigenschaften auf. Die anisotropen elastischen Eigenschaften bewirken, dass die Membran sich nur oder überwiegend in einer Richtung krümmt bzw. wölbt und zumindest näherungsweise die Gestalt eines Zylindermantels annimmt. Alternativ kann die Membran beispielsweise entsprechend geführt sein oder in einen langen und schmalen Rahmen eingespannt sein, um eine im Wesentlichen zylinderförmige Wölbung zu bewirken.

Wenn die Grenzfläche 66 zwischen den beiden Medien 61, 62 von einer geringen Mischbarkeit der beiden Medien 61, 62 herrührt, kann eine zylindrische oder im Wesentlichen zylindrische Wölbung der Grenzfläche 66 insbesondere durch die Form bzw. Gestalt der Kammer 64 (beispielsweise in Projektion auf eine Ebene orthogonal zur optischen Achse 48 lang und schmal) und/oder durch die Gestalt der Grenzlinie zwischen Oberflächen, die von den beiden Medien 61, 62 unterschiedlich benetzt werden, und/oder durch die Anordnung und die Form bzw. Gestalt von Elektroden oder Spulen bewirkt werden.

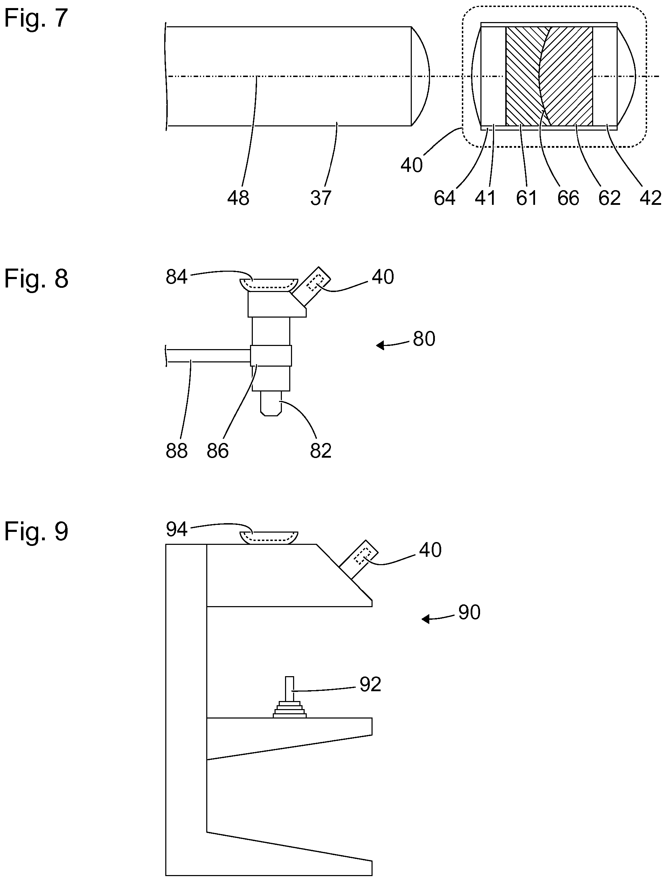

Figur 7 zeigt eine schematische Darstellung eines weiteren Okulars 40, das in einigen Merkmalen und Eigenschaften den oben anhand der Figuren 2 bis 6 dargestellten Okularen und insbesondere dem anhand der Figur 6 dargestellten Okular ähnelt. Die Zeichenebene entspricht den Zeichenebenen der Figuren 1 bis 3, 5 und 6, die Art der Darstellung entspricht derjenigen der Figur 6. Nachfolgend sind Merkmale und Eigenschaften des Okulars 40 beschrieben, in denen sich dieses von dem oben anhand der Figur 6 dargestellten unterscheidet.

Das in Figur 7 dargestellte Okular 40 unterscheidet sich von dem oben anhand der Figur 6 dargestellten Okular insbesondere dadurch, dass die beiden Medien 61, 62 zwischen den sphärischen Linsen 56, 57 angeordnet sind. Die Kammer 64 wird durch ein rohrförmiges Bauteil gebildet, das durch die sphärischen Linsen 56, 57 an beiden Enden verschlossen ist. Die sphärischen Linsen 56, 57 bilden also das Lichteintrittsfenster und das Lichtaustrittsfenster der Kammer 64. Diese Bauweise ist besonders kompakt, weist besonders wenige Oberflächen auf, die einerseits vergütet sein sollen und andererseits verschmutzen können, und weist besonders wenige Grenzflächen auf, an denen unerwünschte Reflexionen erfolgen können.

Bei den anhand der Figuren 1 bis 7 dargestellten Ausführungsformen sind Linsen 41, 42, 56, 57 mit gekrümmten lichtbrechenden Oberflächen vorgesehen. Alternativ oder zusätzlich können andere diffraktive optische Elemente oder Gradientenindexlinsen vorgesehen sein. Insbesondere können Gradientenindexlinsen mit radialem Gradienten die sphärischen Linsen 41, 42 und Gradientenindexlinsen mit axialem Gradienten die Korrekturlinsen 56, 57 ersetzen.

Eine Gradientenindexlinse wird beispielsweise hergestellt, indem ein Glasstab mit ursprünglich homogenem Brechungsindex in einem Salzbad erhitzt wird. Ionen aus dem Salzbad diffundieren in den Glasstab, und Ionen aus dem Glasstab diffundieren in das Salzbad. Dadurch wird ein Teil der Ionen im Glasstab ausgetauscht bzw. ersetzt. Der Austausch von Ionen im Glas verändert den Brechungsindex des Glases. Da nahe der Oberfläche mehr Ionen ausgetauscht werden als im Inneren des Glasstabs, entsteht ein Gradient des Brechungsindexes. In einem kreiszylindrischen Glasstab ist ein von der Zylinderachse nach außen quadratisch oder näherungsweise quadratisch zunehmender Brechungsindex typisch. Abhängig von den ersetzten und den ersetzenden Ionen kann der Brechungsindex erhöht oder verringert werden.

Ein kreiszylindrischer Glasstab mit von innen nach außen zunehmendem oder abnehmendem Brechungsindex wird in der Regel derart verwendet, dass seine Zylinderachse und die optische Achse zusammenfallen. Alternativ kann ein derartiger Glasstab zu einem optischen Bauelement anderer Geometrie verarbeitet werden, insbesondere durch Schleifen oder Sägen. Beispielsweise wird eine Glasplatte mit zwei ebenen und parallelen Oberflächen und nur in einer Richtung variierendem Brechungsindex gefertigt, indem eine Schicht aus dem Glasstab herausgeschnitten wird bzw. derart Material vom Glasstab abgetragen wird, dass diese Schicht übrig bleibt. Die Schicht enthält insbesondere die Zylinderachse. Die beiden ebenen und parallelen Oberflächen sind parallel zur Zylinderachse und dienen als Lichteintrittsfläche bzw. Lichtaustrittsfläche. Die Glasplatte wird insbesondere so verwendet bzw. angeordnet, dass die Zylinderachse und die beiden parallelen Lichtein- und -austrittsflächen jeweils orthogonal zur optischen Achse sind. In dieser Anordnung stellt die Glasplatte eine Gradientenindex-Zylinderlinse dar, die insbesondere die Korrekturlinsen 56, 57 der anhand der Figuren 1 bis 5 dargestellten Ausführungsformen ersetzen oder ergänzen können.

Eine Gradientenindex-Zylinderlinse in Gestalt einer transparenten Platte mit in lediglich einer Richtung variierendem Brechungsindex kann alternativ in anderer Weise hergestellt werden. Insbesondere wird eine Glasplatte mit ursprünglich homogenem Brechungsindex in eine Salz-Schmelze eingetaucht, wobei die Eintauchtiefe eine Funktion der Zeit ist. Dabei sind die beiden ebenen und parallelen Oberflächen der Glasplatte orthogonal zum Flüssigkeitsspiegel der Glasschmelze.

Wenn die Dicke der Glasplatte gering ist, kann Diffusion in Richtung parallel zu den beiden ebenen und parallelen Oberflächen der Glasplatte vernachlässigt werden. In dieser Näherung ist die Anzahl der ausgetauschten Ionen an jedem Ort innerhalb der Glasplatte proportional zur Verweildauer dieses Orts in der Salz-Schmelze.

Beispielsweise bewirkt ein Eintauchen der Glasplatte in die Salz-Schmelze und ein Entnehmen der Glasplatte aus der Salz-Schmelze jeweils mit konstanter Geschwindigkeit einen Brechungsindex, der eine affin-lineare Funktion einer Koordinate ist. Der Gradient des Brechungsindex ist konstant.

Durch ein Eintauchen in die Salz-Schmelze und ein Entnehmen aus der Salz-Schmelze mit nicht-konstanter Geschwindigkeit kann eine nahezu beliebige (monotone) Ortsabhängigkeit des Brechungsindexes erzielt werden. Indem eine rechteckige Glasplatte nacheinander zunächst von einer Kante ausgehend und nach einer Rotation um 180 Grad von der gegenüberliegenden Kante ausgehend und jeweils mit quadratisch variierender Geschwindigkeit in die Salz-Schmelze eingetaucht wird, kann ein Brechungsindex erzielt werden, der von einer Mittelachse ausgehend in beide Richtungen jeweils quadratisch zunimmt. Mit einer anderen Zeitabhängigkeit der Eintauchtiefe und der Eintauchgeschwindigkeit ist eine nahezu beliebige Ortsabhängigkeit des Brechungsindexes realisierbar.

Die oben anhand der Figuren 2 bis 7 dargestellten Okulare sind in oder an Endoskopen einsetzbar, wobei jeweils insbesondere das Okular mit dem Endoskop dauerhaft verbunden ist.

Figur 8 zeigt eine schematische Darstellung eines nicht im Schutzbereich der Anmeldung liegenden Operationsmikroskops 80 mit einem Objektiv 82. In einem Tubus ist ein in gestrichelter Linie angedeutetes Okular 40 angeordnet. Das Okular 40 ist eines der oben anhand der Figuren 2 bis 7 dargestellten Okulare oder weist Merkmale und Eigenschaften auf, die oben anhand der Figuren 2 bis 7 dargestellt wurden. Das nicht im Schutzbereich der Anmeldung liegende Operationsmikroskop 80 kann zwei Okulare 40 aufweisen, um medizinischem Personal einen gleichzeitigen Blick durch das Operationsmikroskop 80 mit beiden Augen und optional sogar Stereoskopie zu ermöglichen.

Ferner umfasst das nicht im Schutzbereich der Anmeldung liegende Operationsmikroskop 80 eine Ablage 84 für eine Brille. Die Ablage 84 ist insbesondere nahe dem oder den Okularen 40 angeordnet, weist die Gestalt einer flachen Schale auf und nimmt eine Brille des medizinischen Personals auf, wenn dieses ohne Brille in das Operationsmikroskop 80 blicken will.

Das nicht im Schutzbereich der Anmeldung liegende Operationsmikroskop 80 weist ferner einen Stativanschluss 86 zur lösbaren mechanischen Verbindung des Operationsmikroskops 80 mit einem Stativ 88 auf. Das Stativ 88 ermöglicht innerhalb vorbestimmter Grenzen eine beliebige oder weitgehend beliebige Anordnung und Orientierung des Operationsmikroskops 80 über einem Operationsfeld. Figur 9 zeigt eine schematische Darstellung einer Steuerungsvorrichtung 90 für einen Chirurgieroboter. Als Chirurgieroboter werden Vorrichtungen bezeichnet, die angetrieben durch Servoantriebe medizinische Eingriffe vornehmen können. Die Steuerung eines Chirurgieroboters erfolgt bislang zumindest zeitweise und zumindest mittelbar durch medizinisches Personal Der Eingriff kann an der Steuerungsvorrichtung 90 durch Blick in ein oder zwei Okulare 40, wie sie oben anhand der Figuren 2 bis 7 dargestellt sind, überwacht werden. Durch die Okulare 40 wird insbesondere ein stereoskopisches Bild erzeugt, das durch eine Kamera an dem Chirurgieroboter oder in seiner Nähe erfasst wird. Zur Steuerung der Servormotoren stehen ein, zwei oder mehr Bedieneinrichtungen 92 zur Verfügung, die beispielsweise als Joysticks oder ähnlich ausgebildet sein können.

Nahe dem oder den Okularen 40 ist eine Ablage 94 für eine Brille vorgesehen. Die Ablage 94 ist insbesondere schalenförmig ausgebildet, um eine Brille von medizinischem Personal, das ohne Brille in das oder die Okulare 40 blicken will, aufzunehmen.

### Bezugszeichen

- 10: Endoskop
- 12: distales Ende des Endoskops 10
- 14: proximales Ende des Endoskops 10
- 16: Schaft des Endoskops 10
- 18: Blickrichtung des Endoskops 10
- 20: Fensterbauteil am distalen Ende 14 des Endoskops 10
- 32: Schwenkprisma am distalen Ende 14 des Endoskops 10
- 34: Objektiv
- 36: Stablinse im Schaft 16 des Endoskops 10
- 37: Stablinse im Schaft 16 des Endoskops 10
- 40: Okular am Endoskop 10 oder am Operationsmikroskop 80 oder an der Steuervorrichtung 90
- 41: erste (distale) sphärische Linse des Okulars 40
- 42: zweite (proximale) sphärische Linse des Okulars 40
- 48: optische Achse des Objektivs 34, der Stablinsen 36, 37 und des Okulars 40
- 56: erste (distale) Korrekturlinse
- 57: zweite (proximale) Korrekturlinse
- 61: erstes Medium mit erstem Brechungsindex
- 62: zweites Medium mit zweitem Brechungsindex
- 64: Kammer für die Medien 61, 62
- 66: Grenzfläche zwischen dem ersten Medium 61 und dem zweiten Medium 62
- 71: erste Bedieneinrichtung zum Einstellen einer Korrekturwirkung der Korrektureinrichtung 56, 57; 61, 62, 66
- 72: zweite Bedieneinrichtung zum Einstellen einer Korrekturwirkung der Korrektureinrichtung 56, 57, 61, 62, 66
- 74: dritte Bedieneinrichtung zum Umschalten zwischen den für Luft und Wasser erforderlichen Korrekturwirkungen der Korrektureinrichtung
- 80: Operationsmikroskop
- 82: Objektiv des Operationsmikroskops 80
- 84: Ablage für eine Brille eines Verwenders des Operationsmikroskops 80
- 86: Stativanschluss zur Befestigung des Operationsmikroskops 80 an einem Stativ 88
- 88: Stativ für Operationsmikroskop
- 90: Steuerungsvorrichtung für einen Chirurgieroboter
- 92: Bedieneinrichtung der Steuerungsvorrichtung 90
- 94: Ablage für eine Brille eines Verwenders der Steuerungsvorrichtung 90

## Patentansprüche

1. Endoskop (10) mit einem Okular (40),
wobei das Okular (40) eine Fokussiereinrichtung (41, 42) zur Positionierung der vom entspannten menschlichen Auge als scharf wahrgenommenen Fläche und zur Korrektur einer Achsen-Ametropie eines Auges eines Verwenders des Okulars (40) aufweist;
wobei das Okular (40) eine Korrektureinrichtung (56, 57; 61, 62, 66) zur stufenlos einstellbaren Korrektur eines Astigmatismus eines Auges eines Verwenders des Okulars (40) aufweist,
wobei die Korrektureinrichtung (56, 57; 61, 62, 66) zwei lichtbrechende Einrichtungen (56, 57), die jeweils in unterschiedlichen Ebenen unterschiedliche Brechwerte aufweisen, umfasst, deren Abstand veränderbar ist, oder die relativ zueinander rotierbar sind,
wobei das Endoskop (10) einen vom Brechungsindex eines Mediums, in dem das distale Ende (12) des Endoskops (10) angeordnet ist, abhängigen Astigmatismus aufweist und die Korrektureinrichtung (56, 57; 61, 62, 66) des Okulars (40) auch eine Korrektur des vom Brechungsindex des Mediums abhängigen Astigmatismus des Endoskops (10) ermöglicht, ferner mit:
einer Bedieneinrichtung (74) zum Ändern des zylindrischen Brechwerts der Korrektureinrichtung (56, 57; 61, 62, 66) um einen vorbestimmten Betrag, der dem Unterschied zwischen dem Astigmatismus des Endoskops (10) bei Anordnung des distalen Endes (12) in Luft und dem Astigmatismus des Endoskops bei Anordnung des distalen Endes in Wasser entspricht.

2. Endoskop (10) nach Anspruch 1, bei dem die Korrektureinrichtung des Okulars (40) eine elektrisch, magnetisch oder mittels hydrostatischen Drucks formbare Grenzfläche (66) zwischen zwei Medien (61, 62) mit unterschiedlichen Brechungsindizes aufweist.

3. Endoskop (10) nach einem der vorangehenden Ansprüche, bei dem die Korrektureinrichtung (56, 57; 61, 62, 66) um die optische Achse (48) des Okulars (40) rotierbar ist.

4. Endoskop (10) nach einem der vorangehenden Ansprüche, wobei das Okular (40) ferner umfasst:
eine Bedieneinrichtung (71) zum Einstellen eines zylindrischen Brechwerts.

5. Endoskop (10) nach einem der vorangehenden Ansprüche, wobei das Okular (40) ferner umfasst:
eine weitere Bedieneinrichtung (72) zum Einstellen der Orientierungen maximalen und minimalen Brechwerts der Korrektureinrichtung ((56, 57; 61, 62, 66).

6. Endoskop (10) nach einem der vorangehenden Ansprüche, wobei die Korrektureinrichtung (56, 57; 61, 62, 66) eine proximale Lichtaustrittsfläche des Okulars (40) bildet.

7. Steuerungsvorrichtung (90) für einen Chirurgieroboter, mit einem Endoskop (10) nach einem der vorangehenden Ansprüche.

## Claims

1. Endoscope (10) comprising an eyepiece (40),
the eyepiece (40) having a focusing device (41, 42) for positioning the area perceived as sharp by the relaxed human eye and for correcting an axial ametropia of an eye of a user of the eyepiece (40);
the eyepiece (40) having a correcting device (56, 57; 61, 62, 66) for the continuously adjustable correction of an astigmatism of an eye of a user of the eyepiece (40);
with the correcting device (56, 57; 61, 62, 66) comprising two light-refracting devices (56, 57) which each have different optical powers in different planes, the spacing between the said devices being modifiable or the said devices being rotatable relative to one another,
the endoscope (10) having an astigmatism that depends on the refractive index of a medium in which the distal end (12) of the endoscope (10) is arranged and the correcting device (56, 57; 61, 62, 66) of the eyepiece (40) also enabling a correction of the astigmatism of the endoscope (10) that depends on the refractive index of the medium, further comprising:
an operating device (74) for changing the cylindrical power of the correcting device (56, 57; 61, 62, 66) by a predetermined value, which corresponds to the difference between the astigmatism of the endoscope (10) when the distal end (12) is arranged in air and the astigmatism of the endoscope when the distal end is arranged in water.

2. Endoscope (10) according to Claim 1, wherein the correcting device of the eyepiece (40) has an interface (66) between two media (61, 62) with different refractive indices, the interface being electrically or magnetically formable or formable by means of hydrostatic pressure.

3. Endoscope (10) according to either of the preceding claims, wherein the correcting device (56, 57; 61, 62, 66) is rotatable about the optical axis (48) of the eyepiece (40).

4. Endoscope (10) according to any one of the preceding claims, the eyepiece (40) further comprising:
an operating device (71) for setting a cylindrical power.

5. Endoscope (10) according to any one of the preceding claims, the eyepiece (40) further comprising:
a further operating device (72) for setting the orientations of maximum and minimum optical power of the correcting device (56, 57; 61, 62, 66).

6. Endoscope (10) according to any one of the preceding claims, the correcting device (56, 57; 61, 62, 66) forming a proximal light exit surface of the eyepiece (40) .

7. Control apparatus (90) for a surgical robot, comprising an endoscope (10) according to any one of the preceding claims.

## Revendications

1. Endoscope (10) comprenant un oculaire (40),
dans lequel l'oculaire (40) présente un dispositif de focalisation (41, 42) pour positionner la surface perçue nettement par l'œil humain détendu et pour corriger une amétropie axiale d'un œil d'un utilisateur de l'oculaire (40) ;
dans lequel l'oculaire (40) présente un dispositif de correction (56, 57 ; 61, 62, 66) pour une correction réglable en continu d'un astigmatisme d'un œil d'un utilisateur de l'oculaire (40),
dans lequel le dispositif de correction (56, 57 ; 61, 62, 66) comprend deux dispositifs réfringents (56, 57) qui présentent respectivement dans des plans différents des puissances optiques différentes et dont l'espacement est variable ou qui peuvent tourner l'un par rapport à l'autre,
dans lequel l'endoscope (10) présente un astigmatisme qui dépend d'un indice de réfraction d'un milieu dans lequel est disposée l'extrémité distale (12) de l'endoscope (10), et le dispositif de correction (56, 57 ; 61, 62, 66) de l'oculaire (40) permet également une correction de l'astigmatisme de l'endoscope (10) qui dépend de l'indice de réfraction du milieu, comprenant en outre :
un dispositif d'actionnement (74) pour modifier la puissance optique cylindrique du dispositif de correction (56, 57 ; 61, 62, 66) d'un montant prédéterminé qui correspond à la différence entre l'astigmatisme de l'endoscope (10) lorsque l'extrémité distale (12) est disposée dans l'air et l'astigmatisme de l'endoscope lorsque l'extrémité distale est disposée dans l'eau.

2. Endoscope (10) selon la revendication 1, dans lequel le dispositif de correction de l'oculaire (40) présente une interface (66) déformable électriquement, magnétiquement ou au moyen d'une pression hydrostatique entre deux milieux (61, 62) ayant des indices de réfraction différents.

3. Endoscope (10) selon l'une quelconque des revendications précédentes, dans lequel le dispositif de correction (56, 57 ; 61, 62, 66) peut tourner autour de l'axe optique (48) de l'oculaire (40).

4. Endoscope (10) selon l'une quelconque des revendications précédentes, dans lequel l'oculaire (40) comprend en outre :
un dispositif d'actionnement (71) pour régler une puissance optique cylindrique.

5. Endoscope (10) selon l'une quelconque des revendications précédentes, dans lequel l'oculaire (40) comprend en outre :
un autre dispositif d'actionnement (72) pour régler les orientations à indice de réfraction maximal et minimal du dispositif de correction (56, 57 ; 61, 62, 66) .

6. Endoscope (10) selon l'une quelconque des revendications précédentes, dans lequel le dispositif de correction (56, 57 ; 61, 62, 66) forme une surface de sortie de lumière proximale de l'oculaire (40).

7. Dispositif de commande (90) pour un robot chirurgical, comprenant un endoscope (10) selon l'une quelconque des revendications précédentes.
